# EUROPEAN PATENT APPLICATION

(11) **EP 4 678 113 A1**
(43) Date of publication of application: **14.01.2026**
(21) Application number: 24315335.0
(22) Date of filing: 08.07.2024
(51) Int. Cl.: A61B 8/00

(54) **CURVED ULTRASOUND FINGERTIP PROBE**

(71) Applicant: Thinkin Tech, 75116 Paris (FR)
(72) Inventor: Ascher, Benjamin, 75116 PARIS (FR)
(74) Representative: Oak & Fox

(57) **Abstract**

An ultrasound fingertip probe comprising:
- a curved ultrasound transducer (30) configured to transmit and receive ultrasounds,
- a C-shaped housing (20) intended to surround a fingertip of a user, the C-shaped housing having a C-shape in a reference plane P, the curved ultrasound transducer being housed in the C-shaped housing (20),
- a finger retainer removably fastened to the C-shaped housing (20) such that the finger retainer limits a tilt of the finger relative to the reference plane when the C-shaped housing (20) surrounds the fingertip.

## Description

### Technical field

The present invention relates to an ultrasound fingertip probe comprising an ultrasound transducer for transmitting and receiving ultrasounds intended to be used to help a physician monitor and carry out a medical diagnostic or a surgical procedure.

### Background

It is known to use ultrasound probes to visualize a fetus in the womb of a pregnant woman to check the fetus's health. For this purpose, the physician holds an ultrasound hand probe and places it on the patient's skin. During the procedure, the hand of the physician holding the probe is occupied, preventing the physician from using that hand for other tasks.

It is also known to use ultrasound fingertip probes to assist a physician during injection procedures. For instance, this type of ultrasound fingertip probe is used to visualize the tissues of the patient's skin to detect veins and arteries, avoiding them when injecting a solution into the patient's skin.

This kind of probe allows normal use of the other fingers of the hand carrying the probe, so that the cheek of the face, for example, can be pinched when injecting there, as is usually done without using a probe.

Another benefit is to let the physician's second-hand free for injection.

There is a need for an improved fingertip ultrasound probe.

### Summary

An aspect of the invention is to limit at least one of the above-mentioned drawbacks.

An aspect of the invention is an ultrasound fingertip probe comprising:
- a curved ultrasound transducer configured to transmit and receive ultrasounds,
- a C-shaped housing intended to surround a fingertip of a user, the C-shaped housing having a C-shape in a reference plane P, the curved ultrasound transducer being housed in the C-shaped housing,
- a finger retainer removably fastened to the C-shaped housing such that the finger retainer limits a tilt of the finger relative to the reference plane when the C-shaped housing surrounds the fingertip.

In at least one embodiment, the finger retainer is a distally opened finger cot removably fastened or secured to the C-shaped housing to receive the finger's distal phalanx when the C-shaped housing surrounds the fingertip.

In at least one embodiment, the distally opened finger cot comprises a guide configured to guide the C-shaped housing along the distally opened finger cot when removably assembling the distally opened finger cot with the C-shaped housing.

In at least one embodiment, the finger retainer comprises two wings facing each other, the reference plane being interposed between the two wings.

In at least one embodiment, the C-shaped housing is configured to remain free from distortion when being placed around the fingertip.

In at least one embodiment, the C-shaped housing comprises:
- a dorsal part configured to face a dorsal part of the fingertip when the C-shaped housing surrounds the fingertip, the dorsal part extending continuously from a first proximal end to a curved part of the C-shaped housing,
- the curved part extending continuously from the dorsal part to a second proximal end and facing a ventral part of the fingertip when the C-shaped housing surrounds the fingertip while the dorsal part faces the dorsal part of the fingertip,
a separation plane being interposed between the first proximal end and the second proximal end, the separation plane being orthogonal to the reference plane and including a first axis of the reference plane along which a longitudinal axis of the finger's distal phalanx extends when the C-shaped housing surrounds the fingertip.

In at least one embodiment, an inner face of the dorsal part forms a straight line in the reference plane, the inner face of the dorsal part being intended to face the finger's the distal phalanx when surrounds the fingertip.

In at least one embodiment, the curved ultrasound transducer is housed in the curved part such that a front curve face of the curved ultrasound transducer forms, in the reference plane, a convex front curve extending from a proximal point to a distal point, towards the dorsal part of the C-shaped housing along a second axis of the reference plane orthogonal to the first axis, a value of an angle formed by a tangent of the convex front curve, at a point of the convex front curve, with the first axis x, continuously increases with a position of the point along the convex front curve from the proximal point to the distal point.

In at least one embodiment, a central portion of an inner face of the curved part forms, in the reference plane, a curve having a first radius of curvature, the inner face of the central portion being interposed, along an axis parallel to the first in the reference plane, between the front face (of the curved ultrasound transducer and a reception volume receiving the fingertip when the C-shaped housing surrounds the fingertip, the inner face of the curved part being intended to face the ventral part of finger's distal phalanx when the C-shaped housing surrounds the fingertip such that the dorsal part of the C-shaped housing faces the dorsal part of the finger's distal phalanx.

In at least one embodiment, a part of an outer face of the ultrasound of the C-shaped part of the ultrasound fingertip probe delimits and encloses the front curved face of the curved ultrasound transducer, and the front curved face of the curved ultrasound transducer form together a continuous convex face that is substantially a partial cylinder, having a generatrix of the cylinder being orthogonal to the reference plane.

In at least one embodiment, the front face of the curved ultrasound transducer is distant along the second axis of the reference plane from a reference point of the curved part. The reference point is the farthest from the separation plane along the second axis among the points of the curved part.

In at least one embodiment,the curved part of the C-shaped housing comprises a ventral part comprises the second proximal end, the ventral part being delimited by an outer of the ventral part and by an inner face interposed between the outer face and a reception volume delimited and surrounded by the C-shaped housing, the dorsal part being delimited by an outer face of the dorsal part and by an inner face of the dorsal part interposed between the outer face of the dorsal part and the reception volume, the outer face of the ventral part being parallel to the outer face of the dorsal part.

In at least one embodiment, the C-shaped housing comprises a notch configured and disposed to receive a distal end of a fingernail protruding, along the first axis, from the pad of the finger's distal phalanx when the C-shaped housing surrounds the fingertip, the dorsal part of the C-shaped housing facing a dorsal part of the finger.

In an embodiment, the ultrasound fingertip probe comprises a wristband comprising a receptacle comprising a wire receptacle receiving an electrical wire or a sheath receiving a plurality of electrical wires in a removable manner, and two slots removably receiving two straps, the wristband being configured to be able to be in an open configuration in which the wristband extends substantially longitudinally and in a second configuration in which the wristband forms a closed loop.

In a second aspect, the invention relates to a kit comprising an ultrasound fingertip probe and a plurality of finger retainers configured to be able to be removably fastened or secured to the C-shaped housing to limit tilt of a longitudinal axis of the finger relative to the reference plane when the C-shaped housing surrounds the fingertip, the plurality of finger retainers comprising a plurality of distally opened finger cots enclosing spaces having different diameters and / or the plurality of finger retainers having two wings facing each other, the two wings of different retainers being separated by respective distinct maximal distances.

### Brief description of the drawings

The above and other objects, features and benefits of the present invention will be more apparent from the following detailed description taken in conjunction with the accompanying drawings, in which:
- Figure 1 shows a schematic side view of a C-shaped part, of a ultrasound fingertip probe this part comprising a C-shaped housing according to a first example and a curved ultrasound transducer housed in a curved part of the C-shaped body,
- Figure 2 shows the surface of the C-shaped part shown in figure 1, in a reference plane P,
- Figure 3 shows a schematic perspective view of the C-shaped part of figure 1,
- Figure 4 shows a schematic frontal view of the C-shaped part of figure 1 showing both the curved ultrasound transducer and the C-shaped housing receiving the curved ultrasound transducer,
- Figure 5 shows a schematic perspective view of a probe according to a first aspect comprising the C-shaped part of figure 1 and a finger retainer according to a first example,
- Figures 6a, 6b and 6c show a schematic perspective view of a finger retainer according to a second example,
- Figure 7 shows a schematic perspective view of the finger retainer of figures 6a, 6b and 6c removably fastened to a C-shaped housing according to a second example, the position of the finger is represented in dotted line,
- Figure 8 shows a schematic perspective view of a wristband comprising a wire and straps receptacle,
- Figure 9 shows a schematic frontal view of the wire and straps receptacle 600, as shown in Figures 8 and 9.

### Detailed description

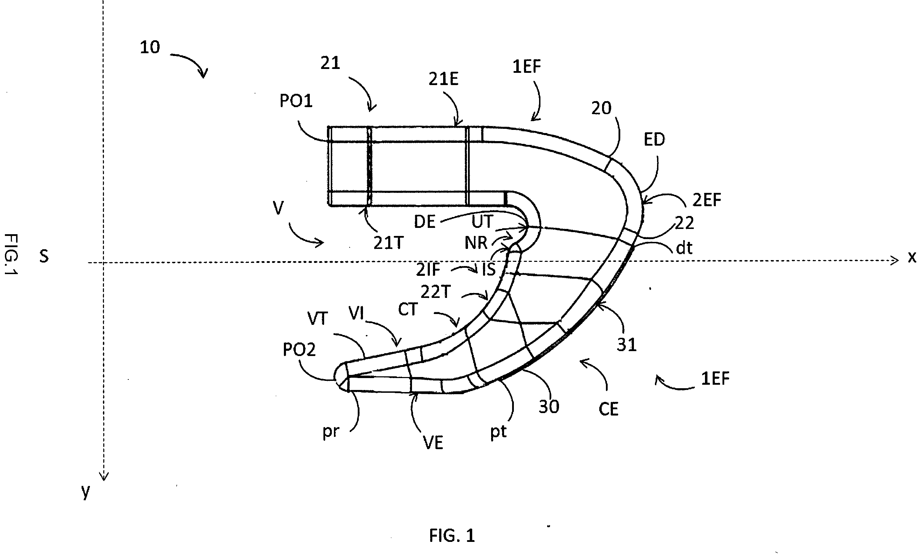

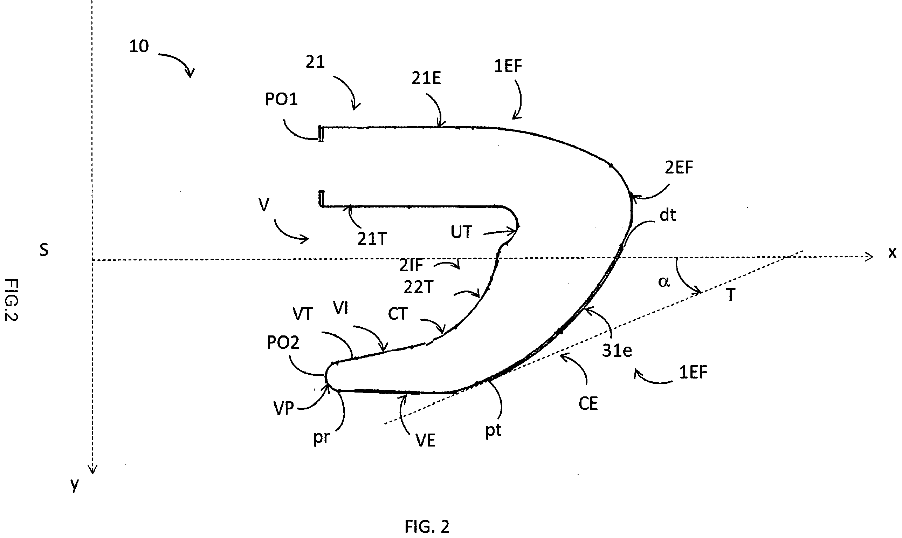

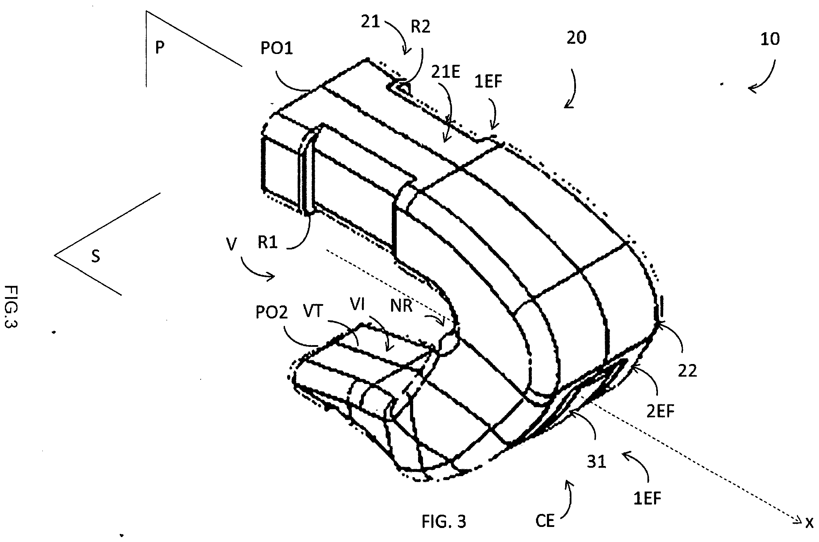

Figure 1 shows a schematic side view of a C-shaped part 10 of a ultrasound fingertip probe according to an aspect. Figure 2 shows the surface of the C-shaped part 10 in a reference plane P which will be defined below. Figure 3 shows a schematic perspective view of the C-shaped part 10 of figure 1.

The C-shaped part 10 of the ultrasound fingertip probe comprises a C-shaped housing 20 and a curved ultrasound transducer 30 housed in or within the C-shaped housing 20 and configured to transmit and receive ultrasounds.

The curved ultrasound transducer 30 is secured to the C-shaped housing 20.

The C-shaped housing 20 is for example made of a plastic material.

In the embodiment of Figure 1, the curved ultrasound transducer 30 may be housed in a recess or in a cavity in the C-shaped housing 20.

It will be appreciated that the benefits of the C-shaped housing 20 are compactness and lightness. Another benefit is easy cleanability and disinfection of this housing, which is essential in medical applications, contrary to known substantially cylindrical bodies having an end closed by a hemispherical cap.

Another benefit is the possibility to removably fasten accessories to the C-shaped housing 20 to ensure perfect adhesion between the distal phalanx (usually called P3) of the finger and the C-shaped housing 20.

Another benefit is the possibility to removably fasten accessories to the C-shaped housing 20 to maintain the longitudinal axis of the finger's distal phalanx aligned with a predetermined axis fixed with respect to the C-shaped housing 20.

The C-shaped housing 20 has a C-shape in a reference plane P parallel to the plane of Figure 2 and shown in Figure 3.

The reference plane P is fixed relative to the C-shaped housing 20.

The C-shaped housing 20 is configured to surround a finger's distal end of a user.

Therefore, the C-shaped housing 20 delimits and surrounds a reception volume V (i.e. a reception space) intended to receive the finger's distal phalanx of the user.
- This configuration allows the C-shaped part 10 of the probe to be secured around the distal end finger of the user.

Beneficially, in one or more embodiment, the C-shaped housing 20 is shaped and dimensioned to fit or closely fit a finger's distal end of a user. The dimensions and shape of such a C-shaped housing are disclosed below.

In other words, in one or more embodiments, the volume enclosed by the C-shaped housing 20 is dimensioned and shaped to be substantially complementary to the distal end of a finger of predetermined forms and dimensions.

When the finger of the user presses the C-shaped housing 20 to press the curved ultrasound transducer against the surface of the skin of a patient, the C-shaped housing stays in place on the tip of the finger.

In this configuration, the fingertip rests or abuts against the C-shaped housing 20 along the longitudinal axis.

The C-shaped housing 20 comprises an inner face 2IF delimiting the reception volume V.

The inner face 2IF of the C-shaped housing 20 is intended to face the finger's distal phalanx when the C-shaped housing 20 surrounds the fingertip.

Beneficially, this inner face 2IF is the inner face of the ultrasound C-shaped part 10 of the probe.

The C-shaped housing 20 further comprises an outer face 2EF surrounding the inner face 2IF of the C-shaped housing 20.

Each of the outer face 2EF and the inner face 2IF is generally C-shaped.

As a result, each of the outer face 2EF and the inner face 2IF generally forms a C in the reference plane P.

Beneficially, the inner face 2IF of the C-shaped housing is substantially a part of a first cylinder having a generatrix orthogonal to the reference plane P and the outer face 2EF of the C-shaped housing is substantial part of a second cylinder having a generatrix orthogonal to the reference plane P.

A benefit of the disclosed configuration is an easy manufacturing of the C-shaped housing.

Alternatively, the inner face 2IF and/or the outer face 2EF may be convex in a plane or in planes orthogonal to the reference plane P comprising the first axis x.

The C-shaped housing 20 comprises a dorsal part 21 and a curved part 22.

The dorsal part 21 is intended to face a dorsal part of the fingertip when the C-shaped housing 20 surrounds the fingertip.

By dorsal part of the fingertip, it is meant the face of the finger's distal phalanx comprising the finger's nail.

By the ventral part of the fingertip, it is meant the pad of the finger's distal phalanx.

The dorsal part 21 extends continuously from a first proximal end PO1 to the curved part 22.

The curved part 22 extends continuously from the dorsal part 21 to a second proximal end PO2 facing a ventral part of the fingertip when the C-shaped housing 20 surrounds the fingertip such that the dorsal part 21 faces the dorsal part of the finger's distal phalanx.

The dorsal part 21 and the curved part 22 form the C-shape of the housing 20, including in the reference plane P.

The first proximal end PO1 is separated from the second proximal end PO2 by a separation plane S orthogonal to the reference plane P and comprising a first axis x of the reference plane P along which a longitudinal axis of the distal phalanx is intended to extend when the C-shaped housing 20 surrounds the fingertip in such a way that the dorsal part 21 faces the dorsal part of the distal phalanx of the finger and the curved part 22 faces the ventral part of the distal phalanx of the finger.

In other words, the separation plane S is interposed between the first proximal end PO1 and the second proximal end PO2.

The separation plane S is fixed relative to the C-shaped housing 20.

In the description below, by the C-shaped housing 20 is placed around the fingertip or is in place, it is meant that the C-shaped housing 20 surrounds the fingertip of the user such that the dorsal part 21 of the C-shaped housing 20 faces the dorsal part of the finger, for example the dorsal part of the finger's distal phalanx and the ventral part 22 of the C-shaped housing 20 faces the ventral part of the finger, for example of the finger's distal phalanx.

Beneficially, the C-shaped housing 20 is configured such that the second proximal end PO2 faces a ventral part of the finger, for example of the finger's distal phalanx, and the first proximal end PO1 faces a dorsal part of the finger, for example of the finger's distal phalanx.

Beneficially, in an embodiment, a length of the reception volume V measured along the first axis x is comprised between a minimal length and a maximal length. The minimal length is between 5 mm and 15 mm and the maximal distance is between 30 mm and 50 mm and the maximal length is for example comprised between 30 mm and 40 mm or between 40 mm and 45 mm.

This length is for example the maximal distance among a first distance between the first proximal end PO1 and a distal extremity DE of the inner face 2IF measured along the first axis x and a second distance between the second proximal end PO2 and the distal extremity DE of the inner face 2IF measured along the first axis x.

In an embodiment, the first distance is equal to the second distance

In another embodiment, the first distance is greater than the second distance. A benefice is the limitation of the obstacle to the movement of the second phalanx P2 relative to the third phalanx P3. Beneficially, in an embodiment, a length of the C-shaped housing along the first axis x is comprised between 15 mm and 60 mm, for example between 15 mm et 40 mm.

This length is for example the maximal distance among a first distance between the first proximal end PO1 and a distal extremity ED of the C-shaped housing 20 measured along the first axis x and a second distance between the second proximal end PO2 and the distal extremity ED of C-shaped housing 20 measured along the first axis x.

A benefit of the disclosed configuration is easy mobility of the second phalanx P2 in relation to the first phalanx P1 (proximal phalanx) and of the third phalanxP3 (distal phalanx) in relation to the second phalanx P2, the second phalanx P2 connecting the first phalanx P1 to the third phalanx P3.

The curved ultrasound transducer 30 comprises a front curved face 31 intended to be placed in direct physical contact with the patient's skin.

The front curved face 31 is the only face of the curved ultrasound transducer 30 which is intended to be placed in direct physical contact with the patient's skin.

By acquiring ultrasounds, it is meant transmitting ultrasound signals toward a region of interest of a patient's body, receiving ultrasounds backscattered from the region of interest and generating electrical signals from the ultrasound signals reflected by the region of interest.

The electrical signals are intended to be used to reconstruct an image, for example a bidimensional image,, of the region of interest.

The disclosed configuration of a transducer with a curved front face 31 is particularly beneficial as it allows for a full-contact surface of the transducer's front face 31 with the patient while providing access to relatively hard-to-reach areas such as concave and convex surfaces of the face.

Despite the limited size of the contact surface, the size of the produced image is not diminished. It remains fully comparable to that of a larger linear transducer bar.

The curved ultrasound transducer 30 is housed in the curved part 22 of the C-shaped housing 20 such that the front curved face 31 of the curved ultrasound transducer 30 forms, in the reference plane P, a convex front curve 31e referenced in Figure 2, extending from a proximal point pt to a distal point dt toward the dorsal part 21 of the C-shaped housing 20 along a second axis y of the reference plane P orthogonal to the first axis x.

The second axis y is fixed relative to the C-shaped housing 20.

According to an embodiment, a value of an acute angle α formed by a tangent T to the convex front curve 31e, at a predetermined point of the convex front curve 31e, with the first axis x, continuously increases with the position of that point along the convex front curve 31e from the proximal point pt to the distal point dt.

According to an embodiment, a distance between the second proximal end PO2 and the convex front face curve 31e along the longitudinal axis continuously increases from the proximal point pt to the distal point dt.

Figure 2 shows the angle α between the tangent of the curve 31e and the axis x at the distal point pt of the convex front curve 31e formed by the front curved face 31 of the curved ultrasound transducer 30 in the reference plane P.

A benefit of the disclosed configuration is the reduction of finger fatigue when pressing the curved ultrasound transducer 30 against the patient's skin, with the C-shaped housing 20 surrounding the fingertip. This is because the finger rests on the skin in the same natural position it would assume without the transducer. The reaction from the skin's surface on the finger forms an angle with the longitudinal axis of the finger (or distant phalanx) in the reference plane P that is neither 0° nor 90°.

Beneficially, the curved ultrasound transducer 30 is positioned in or within the housing 20 such that the front curved face 31 of the curved ultrasound transducer 30 is intended to be adjacent to the finger pad of the distal phalanx of the finger along the first axis x in the reference plane P, when the C-shaped housing 20 is placed around the fingertip. This configuration contributes significantly to the physician's comfort during use.

Alternatively, the angle α continuously increases from a minimal value to a maximal value from the proximal point pr to the distal point pt.

Beneficially, in an embodiment, the minimal value is positive or null.

Beneficially, in an embodiment, the maximal value is less than 90° or 80° or is equal to 90°.

Alternatively, the front curved face 31 extends continuously in the reference plane P, from the proximal point to a distal point pt and form the distal point pt to another proximal point such that the angle α varies from an acute value to an obtuse value.

The radius of curvature of the curve 31e formed by the front curved face 31 of the curved ultrasound transducer 30 in the reference plane P is comprised between 10 mm and 50 mm or between 20 mm and 40 mm or between 25 mm and 35 mm.

For example, in an embodiment, the radius of curvature of the curve is equal to 30 mm.

In the example shown in Figures 1 to 3, the curve 31e has a single radius of curvature.

In this configuration, the convex front curve 31e is circular.

Alternatively, in another embodiment, a radius of curvature of the convex front curve 31e may vary along this curve 31e.

### Distortion of the C-shaped housing

Beneficially, in an embodiment, the C-shaped housing 20 is configured to remain free from distortion when placing the C-shaped housing around the fingertip.

Beneficially, in an embodiment, the C-shaped housing 20 is configured to remain free from distortion during use.

In other words, the C-shaped housing 20 maintains its shape without distortion when being placed around the fingertip, when the finger is removed from the reception volume V, and when the finger presses the front curved face 31 of the curved ultrasound transducer 30 against the patient's skin during ultrasound acquisition.

A benefit of this configuration is the relatively fixed positioning and orientation of the finger's distal phalanx relative to the C-shaped housing 20, especially when the shape of the distal phalanx in the reference plane P is complementary to the shape of the C-shaped housing 20 in the reference plane P.

This feature facilitates easier interpretation of the images generated from ultrasound signals received during acquisition.

### Stability of the curved ultrasound transducer

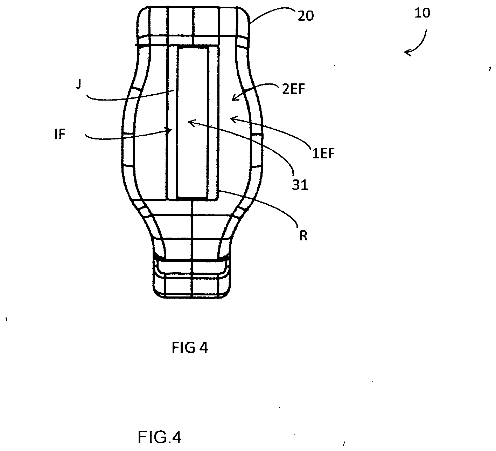

Figure 4 shows a schematic frontal view of the curved ultrasound transducer 30 and of the C-shaped housing 20 receiving the curved ultrasound transducer 30.

The outer face 1 EF of the C-shaped part 10 of the probe comprises the outer face 2EF of the C-shaped housing 20 and the front curved face 31 of the curved ultrasound transducer 30.

Beneficially, in an embodiment, as shown in Figure 4, a part of the outer face 1EF of the C-shaped part 10 of the fingertip probe forms a closed frame delimiting and enclosing the front curved face 31 of the curved ultrasound transducer 30.

Beneficially, in an embodiment this closed frame and the front curved face 31 of the curved ultrasound transducer 30 together form a continuous convex face that is substantially a partial cylinder with a generatrix orthogonal to the reference plane P.

A benefit of this design is the stabilization of the curved ultrasound transducer 30 when pressing it against the patient's skin, allowing for the generation of high-quality images.

Another benefit of this design is the reduction of finger fatigue when pressing the curved ultrasound transducer against the patient's skin.

Additionally, with the disclosed design, the physician can roll the curved transducer 30 on the surface of the skin of a patient to vary the orientation of the curved transducer 30 relative to the skin.

In the example shown in Figures 1 to 4, the aforementioned cylinder is a cylinder of revolution.

Alternatively, this cylinder is distinct from a cylinder of revolution.

Alternatively, the closed frame and the front curved face 31 of the curved ultrasound transducer 30 together form a continuous face that is convex in a plane orthogonal to the reference plane P.

Beneficially, in an embodiment as shown in Figures 3 and 4, the closed frame comprises a first closed frame surrounding the curved ultrasound transducer 30 that is a part of the outer face 2EF of the housing 20.

This design participates in the stability of the probe when pressed against the patient's skin.

In an example, as shown in Figure 4, the closed frame comprises an intermediate face IF of at least one junction element J that extends from the front curved face 31 of the curved ultrasound transducer 30 and the outer face 2EF of the housing 20.

A benefit of this design is its ability to bridge the gap between the outer face 2EF of the housing 20 and the curved ultrasound transducer 30. This feature facilitates the installation of the curved ultrasound transducer 30 into the housing 20. Moreover, it accommodates potential dimensional inaccuracies of both the curved ultrasound transducer 30 in the housing 20 and the recess R in the housing 20 that receives the curved ultrasound transducer 30. This tolerance for slight variations in dimensions enhances the overall flexibility and ease of assembly of the device.

In the non-limiting example of figures 3 and 4, the junction element J forms a closed frame surrounding the front curved face 31 of the curved ultrasound transducer 30.

In another example, the closed outer face 2EF of the housing 20 is contiguous to, the front curved face 31 of the curved ultrasound transducer 30.

By contiguous parts, it is meant directly adjacent parts with no gap between them.

In this example, the junction element forms an open frame.

Alternatively, at least two junction elements are arranged around the front curved face 31 of the curved ultrasound transducer 30.

Alternatively, the closed outer face 2EF of the housing 20 is contiguous to the front curved face 31 of the curved ultrasound transducer 30 along the entire circumference of the front curved face 31 of the curved ultrasound transducer 30.

Beneficially, in an embodiment, the front curved face 31 of the curved ultrasound transducer 30 is adjacent, along the first axis x in the reference plane P, to the volume V.

Beneficially, in an embodiment, the front curved face 31 of the curved ultrasound transducer 30 is distant (i.e. is positioned at a distance) along the second axis y from the reference plane P (which is orthogonal to the first axis x) from a reference point pr of the curved part 22.

This reference point pr is the point of the curved part 22 that is farthest from the separation plane S along the second axis y.

A benefit of this configuration is that it helps to reduce finger fatigue when pressing the curved ultrasound transducer 30 against the patient's skin.

Alternatively, in another configuration, the distance between the front curved face 31 of the curved ultrasound transducer 30 and the reference point pr, measured along the second axis y, may be zero.

### Outer face of the C-shaped housing

Beneficially, in an embodiment, the dorsal part 21 of the C-shaped housing 20 is bounded along the second axis y by an outer face 21E of the dorsal part, which forms part of the outer face 1EF of the C-shaped part 10 of the fingertip probe and by an inner face 21T, which forms part of the inner face 1EF of the C-shaped part 10 of the fingertip probe.

Beneficially, in an embodiment, the outer face 21E of the dorsal part 21 of the C-shaped housing 20 is substantially parallel to the first axis x in the reference plane P.

In other words, the outer face 21E of the dorsal part 21 of the C-shaped housing 20 forms a curve in the reference plane P. This curve is a straight line substantially parallel to the first axis x.

A benefit of this design is the compactness of the ultrasound probe 10, which allows imaging of hard-to-access parts of the skin.

For example, the outer face 21E is flat.

The curved part 22 of the C-shaped housing 20 includes a ventral part VT comprising the proximal end PO2. This ventral part VT is bounded, along the second axis y of the reference plane P, by an outer face VE of the ventral part VT being part of the outer face 1 EF and by an inner face VI of the ventral part VT being part of the inner face 21F.

The inner face VI and the outer face VE delimit the ventral part VT along the second axis y.

The inner face VI of the ventral part VT is connected to the outer face VE of the ventral part VT by a convex proximal surface VP comprising the proximal end PO2.

Beneficially, the outer face VE of the ventral part VT of the C-shaped housing is substantially parallel, for example parallel, to the second axis y in the reference plane P. By "substantially", it is meant that an angle between the outer face VE and the second axis y may be less than 5 degrees, such as for example less than 3 degrees of than 2 degrees or one degree.

This design contributes to the compactness of the ultrasound probe, allowing imaging of hard-to-access parts of the skin.

For example, the outer face VE is flat.

For example, the outer face VE is substantially parallel, for example parallel, to the separation plane S.

By "substantially", it is meant that an angle between the outer face VE and the separation plane P may be less than 5 degrees, such as for example less than 3 degrees or than 2 degrees or one degree.

This feature enhances the compactness of the ultrasound probe. Another benefit is the stability of the ultrasound probe when placed on a table via the outer face VE of the ventral part VT of the C-shaped housing 20.

Beneficially, in an embodiment, the outer face VE of the ventral part of the C-shaped housing is substantially parallel to the outer face 21E of the dorsal part 21 of the C-shaped housing 20.

By "substantially", it is meant that an angle between the outer face VE and the outer face 21E may be less than 5 degrees, such as for example less than 3 degrees or than 2 degrees or one degree. This feature enhances the compactness of the ultrasound probe.

Alternatively, the outer face 21E of the dorsal part 21 of the C-shaped housing 20 is tilted relative to the outer face VE of the ventral part VT of the curved part 22 at least in the reference plane P.

Alternatively, the outer face VE of the ventral part VT of the curved part 22 is curved in the reference plane P.

For example, the outer face VE of the ventral part VT of the curved part 22 forms a circular curve in the reference plane P.

The width of the C-shaped body is measured along a third axis orthogonal to both the first axis x and the second axis y.

Beneficially, in an embodiment, the outer face VE of the ventral part VT comprises the reference point pr.

The curved part 22 of the C-shaped housing 20 comprises a central part CE that extends continuously from the ventral part VT to the dorsal part 21.

### Inner face of the C-shaped housing.

The inner face 2IF of the C-shaped housing 20 is designed to face the distal phalanx of the finger when the housing is placed around the fingertip.

Beneficially, in an embodiment, the inner face 2IF of the C-shaped housing 20 is configured to substantially fit, at least in the reference plane P, the fingertip or the fingertip's distal phalanx when the C-shaped housing is in place.

In other words, the inner face 2IF of the C-shaped housing 20 is configured to have, at least in the reference plane P, a shape substantially complementary to the shape of a fingertip in a sagittal plane of the finger, for example of the finger's distal phalanx end.

This design makes the C-shaped part 10 of the fingertip probe 10 ergonomic and relatively stable on the finger.

Advantageously, the C-shaped body 20 is shaped and dimensioned such that the dorsal part 21 is substantially complementary, in the reference plane P, to the dorsal part of the distal phalanx, and that the curved part 22 is substantially complementary to the ventral part of the distal phalanx when the C-shape housing is in place.

The inner face 21T of the dorsal part 21 of the C-shaped housing 20 is beneficially substantially parallel to the first axis x in the reference plane P.

By "substantially", it is meant that an angle between the inner face 21T and the first axis x may be less than 5 degrees, such as for example less than 3 degrees or less than 2 degrees or one degree.

A benefit of this design is that the inner face 21T of the dorsal part 21 of the C-shaped housing substantially fits the curvature of the dorsal part of the finger's distal phalanx (i.e. the fingernail) surrounded by the C-shaped housing, in the reference plane P.

For example, inner face 21T is flat.

Beneficially, in an embodiment, the inner face 21T of the dorsal part 21 of the C-shaped housing is substantially parallel to the separation plane S.

By "substantially", it is meant that an angle between the inner face 21T and the separation plane S may be less than 5 degrees, such as for example less than 3 degrees.

Beneficially, in an embodiment, the inner face 21T of the dorsal part 21 of the C-shaped housing 20 is substantially parallel to the outer face 21E of the dorsal part 21 of the C-shaped housing. This is typically the case when the C-shaped housing is substantially part of a cylinder.

The inner face 22T of the curved part 22 of the C-shaped housing 20 continuously extends from the inner face 21T of the dorsal part 21 to the second proximal end PO2.

The inner face VI of the ventral part VT is beneficially flat.

More generally, the inner face VI of the ventral part VT forms a straight-line curve in the reference plane P.

Beneficially, in an embodiment, the straight-line curve forms an acute angle comprised between 2° et 10°, for example between 3° and 7° with the first axis x.

Beneficially, in an embodiment, the curved part 22 comprises a central portion CE having an inner face CT being part of the inner face 2IF that partially delimits the reception volume V and forms, in the reference plane P, a curve having a first radius of curvature.

Beneficially, in an embodiment, the inner face CT of the central portion CE and the front face 31 of the curved ultrasound transducer 30 are distributed along at least one axis to the first axis x in the reference plane P.

In other words, the central portion CE comprises an inner face CT interposed between the front face 31 of the curved ultrasound transducer and the reception volume V along at least one axis to the first axis x in the reference plane P.

Beneficially, in an embodiment, the inner face CT of the central portion CE and the inner face VI of the ventral part VT together form a continuous concave face.

Beneficially, in an embodiment, the radius of curvature of the inner face CT of the central portion CE in the reference plane P is smaller than a radius of curvature of the convex front curve 31e. It permits enclosing as closely as possible the distal phalanx's pad.

The radius of curvature of the inner face CT of the central portion CE in the reference plane P is beneficially comprised between 8 mm and 15 mm.

It allows a close fit between the pad of the first phalanx P3 and the C-shaped body 20.

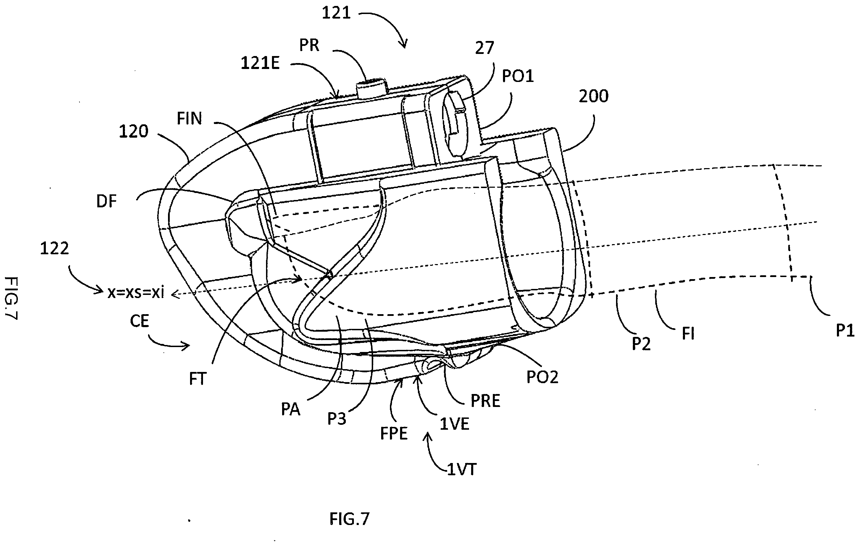

Beneficially, in an embodiment the C-shaped housing comprises a notch NR configured and disposed to receive a distal end of a fingernail FIN (shown in figure 7) protruding, along the first axis x, from the pad PA of the finger's FI distal phalanx P3 when the C-shaped housing 20 surrounds the fingertip.

Beneficially, in an embodiment, the inner face 22T of the curved part 22 includes an upper inner face portion UT. This portion is positioned, along the second axis y, between the inner face CT of the central portion CE and the inner face 21T of the dorsal part 21 of the housing 20. It forms a notch NR designed to accommodate the distal end of a fingernail protruding from the pad PA of the distal phalanx P3, along the first axis x, when the C-shape housing surrounds the fingertip.

Beneficially, in an embodiment, the upper inner face portion UT includes the distal end DE of the inner face 22T of the curved part 22 or, more generally, of the inner face 2IF of the C-shaped housing 20.

Beneficially, the distal end DE is positioned at a distance from the inner face CT of the central portion CE along the first axis x.

This design accommodates the fact that a fingernail protruding from the pad of the finger's distal phalanx (P3) forms the fingertip.

The fingernail can easily be housed in the notch NR.

Beneficially, the upper inner face portion UT is contiguous with the inner face 21T of the dorsal portion 21.

In an example, the upper inner face portion UT is circular in the reference plane P.

Beneficially, in an embodiment, a radius of curvature of the inner face portion UT, in the reference plane P, is smaller than the radius of curvature of the inner face CT of the central portion CE.

Beneficially, in an embodiment, the radius of curvature of the inner face portion UT, in the reference plane P, is comprised between 1 mm and 3 mm.

Alternatively, the inner face portion UT is configured such that the notch NR is elongated along the first axis x.

Beneficially, in an embodiment, the inner face CT of the central portion CE is contiguous with the inner face 21T of the dorsal part 21.

Beneficially, in an embodiment, the inner face CT of the central portion CE is connected to the inner face portion UT by a convex intermediate surface IS, convex in the reference plane P.

Beneficially, in an embodiment, a maximal height of the volume V along the second axis y is comprised between 8 mm and 25 mm, for example between 8 mm and 18 mm or between 8 mm and 20 mm.

It allows closely fitting of the C-shaped housing 20 with the fingertip of many sizes of fingertips.

Beneficially, in an embodiment, a width of the C-shaped housing along an axis orthogonal to the reference plane P is comprised between 15 mm and 25 mm.

For example, the width is equal to 18 mm.

### Finger retainers

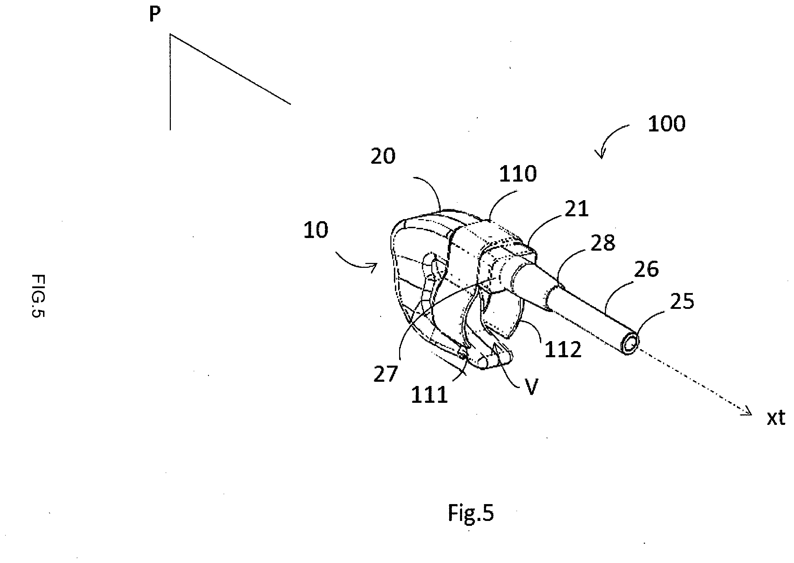

Figure 5 shows a schematic perspective view of an ultrasound probe 100 according to an embodiment.

The ultrasound probe comprises the C-shaped part 10 of the fingertip probe 10 and a finger retainer 110 according to a first example.

Alternatively, the ultrasound probe is devoid of retainers.

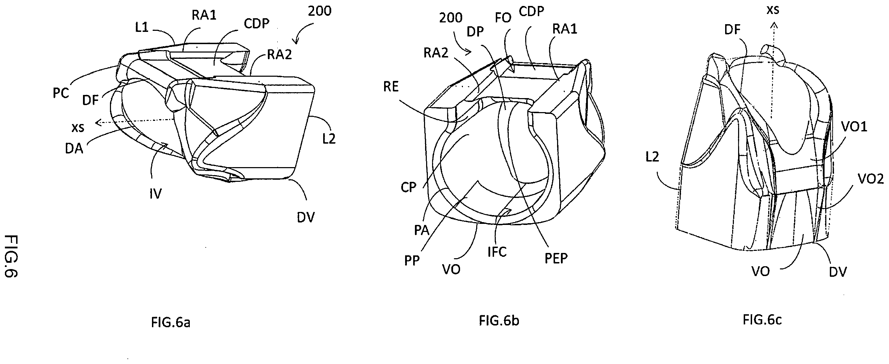

Figure 6&, 6b and 6c show a schematic perspective view of a finger retainer 200 according to a second example.

The finger retainer 110, 200 is configured to limit a tilt, that is to say a magnitude of a tilt, of the finger's longitudinal axis xi, or of at least the distal phalanx's longitudinal axis, relative to the reference plane P when the C-shaped housing 20 surrounds the fingertip and the finger retainer 110, 200 is fastened to the C-shaped housing 20.

Beneficially, this limitation occurs when the C-shaped housing 20 is placed around the fingertip, the fingertip resting against the inner face 22T of the curved part 22 along the first axis or along an axis parallel to the first axis x.

A benefice is a limitation of the movement of the finger's longitudinal axis xi relative to the C-shape housing around the P plane without limiting the natural movement of the finger and its phalanges.

The finger retainer 110, 200 and the C-shaped housing 20 are removably attached to each other. This feature allows for easy cleaning and disinfection of both the C-shaped housing and the finger retainer 110, 200.

Beneficially, the finger retainer 110, 200 is configured such that the part of the fingertip rests or abuts against the inner face 22T of the curved part 22 along the first axis or along an axis parallel to the first axis x is in direct physical contact with the inner face 22T of the curved part 22 when the retainer 110, 200 is fastened to the C-shaped housing 20 surrounding the finger.

This feature allows for easy cleaning and disinfection of both the C-shaped housing and the finger retainer 110, 200.

Beneficially, in an embodiment, the finger retainer 110, 200 and the C-shaped housing 20 are configured to be removably attached to each other to form a snap-fit assembly.

Beneficially, in an embodiment, the finger retainer 110, 200 and the C-shaped housing 20 are configured to be removably snap-fit assembled through the elastic deformation of the finger retainer 110.

Consequently, the finger retainer 110, 200 and the C-shaped housing 20 are configured to be detached from each other by the elastic deformation of the finger retainer 110.

An aspect of the invention also relates to a kit comprising a C-shaped housing and a plurality of finger retainers 110, 200 of different sizes.

These retainers delimit and surround respective volumes with different maximal widths along a third axis orthogonal to both the first axis x and the second axis y when removably attached to the C-shaped housing 20.

This allows a physician to choose the finger retainer that best fits the diameter of his finger.

Beneficially, in an embodiment, the maximal width is comprised between 10 mm or 11 mm and 20 mm, for example between 15 mm and 18 mm.

### Finger retainer with lateral retaining wings

In the example shown in Figure 5, the finger retainer 110 comprises two lateral retaining wings 111, 112 that face each other.

The reference plane P is interposed between the retaining wings 111, 112 and the lateral retaining wings are intended to face the distal phalanx of the finger when the C-shaped housing 20 is placed around the fingertip.

In other words, each of the two retaining wings 111, 112 face the volume V.

The two retaining wings 111, 112 are configured to squeeze or pinch or enclose the physician's finger.

This limits tilt of the finger relative to the reference plane P or ensures that the longitudinal axis of the finger's distal phalanx remains in a predetermined orientation relative to a rotation axis of the reference plane P when the physician presses the curved ultrasound transducer 30 against the skin a patient. This rotation axis is parallel to the first axis and fixed to the C-shaped housing.

Beneficially, the wings 111, 112 are flexible such they can be in a resting position and in a flexed position in which they are flexed such they are further apart from each other along the third axis.

This allows an adaptation of the finger retainer to different fingers' diameters and to enclose fingers of different diameters.

The rotation axis is for example the first axis x.

Beneficially, in an embodiment, the finger retainer 110 is attached to the dorsal part 11 of the C-shaped housing 20.

In the example shown in Figure 1, the dorsal part 21 of the S-shaped housing 20 comprises two recesses R1, R2, with the reference plane P being situated between the two recesses R1 and R2, alongside the finger retainer 110.

Beneficially, in an embodiment, the finger retainer 110 has a general U-shape, with the two lateral wings 111 and 112 facing each other and a dorsal part 113 extending between the first wing 111 to the second wing 112.

Beneficially, in an embodiment, the finger retainer 110 and the C-shaped housing 20 are configured to be removably fastened to each other via a snap-fit assembly, which involves the elastic deformation of the finger retainer 110. The first wing is housed in the first recess R1, with a second part of the first wing facing the volume V. Similarly, a first part if the second wing 112 is housed in the second recess R2, with a second part of the second wing facing the volume V. The dorsal part 113 of the finger's retainer 110 rests against the outer face of the dorsal part.

The finger retainer is elastically deformed to spread the two wings apart from each other for attachment and detachment from the C-shaped housing 20.

Beneficially, in an embodiment, each one of the first wing 111 and the second wing 112 have a circular part.

Beneficially, in an embodiment, the radius of curvature of the circular part of the first wing 111 is equal to the radius of curvature of the circular part of the second wing 112.

Beneficially, in an embodiment, the different finger retainers 110 of the kit differ from each other in that the radius of curvature of the circular part of their wings is distinct.

In other words, he radius of curvature of the circular part of the wings of the different finger retainers are different from each other.

More generally, the two wings of the different finger retainers 110 are separated by different respective maximal distances.

### Distally opened finger cot

Figures 6a, 6b and 6c show a schematic perspective view of a distally opened finger cot 200, which serves as a finger retainer.

The distally opened finger cot 200 can be seen as a distally opened thimble.

The distally opened finger cot 200 has a distal aperture DA and a proximal aperture PA.

Figure 7 shows a schematic perspective view of the distally opened finger cot 200 removably fastened to a C-shaped housing 120 according to a second example. In figure 7, the C-shaped housing 120 surrounds the fingertip FT of the finger Fl.

The C-shaped housing 120 shown in figure 7 differs from the C-shaped housing 20 according to the first example only in that the dorsal part 121 of the C-shaped housing 120 differs from the dorsal part 21 of the C-shaped housing 20 and in that the ventral part 122 of the C-shaped housing 120 differs from the ventral part 22 of the C-shaped housing 20.

The dorsal part 121 of the C-shaped housing 120 differs from the dorsal part 21 of the C-shaped housing 20 only in that the outer face 121E of the dorsal part 121 of the C-shaped housing 120 comprises a flat portion FP, and a protrusion PR protruding from the plane of the flat portion FP.

Alternatively, the C-shaped housing 120 comprises as straight-line portion in the reference plane P and a protrusion PR protruding from the straight line in the reference plane P.

The protrusion PR may be provided in any variant or examples the outer faces of the dorsal part 21 described above.

This protrusion is for example intended to be received in an aperture of a finger retainer having lateral wings 110.

The finger retainer may participate in the fastening of the finger retainer to the C-shaped housing.

The curved part 122 of the C-shaped housing 120 differs from the curved part 22 of the C-shaped housing 20 only in that the outer face 1VE of the ventral part 1VT of the C-shaped housing 120 comprises a slot PRE.

In other words, the outer face 1VE of the ventral part 1VT of the C-shaped housing 120 comprises a flat portion FPE, and a slot PRE in the flat portion FPE. The slot extends PRE continuously along the entire width of the C-shaped body 120.

The slot PRE may be provided in any one of the examples of variants of the outer face VE of the ventral part VT described above.

An elastic can be inserted into the slot PRE to enclose the finger surrounded by the C-shaped housing by enclosing the dorsal and ventral parts of the C-shaped housing to act as a finger retainer as described below.

In this example, the elastic acts as a finger retainer removably attached to the finger.

In an embodiment, the ultrasound fingertip probe comprises a C-part comprising a C-shaped housing 120, 20 and an ultrasound transducer 30 housed in the C-shaped housing 120, 20 and fastened to the C-shaped housing 120 and at least one finger retainer, for example more than one finger retainer, removably fastened to the C-shaped housing among an elastic, a finger retainer with lateral retaining wings and a distally opened finger cot.

For example, the ultrasound fingertip probe comprises an elastic removably attached to the C-shaped housing 120, an elastic received in the slot PRE and enclosing the dorsal and ventral parts of the C-shaped housing 120 and a distally opened finger cot 200 received in the reception volume C and fastened to the C-shaped housing 20.

Any one of the variants of the C-shaped housing 20 described above is applicable to the C-shaped housing 120.

Beneficially, in an embodiment, the C-shaped housing 20,120 and the distally opened finger cot 200 are configured such that the distally opened finger cot 200 is intended to be removably fastened to the C-shaped housing such that the distally opened finger cot is fixed relative to the C-shaped housing 20, 120.

In this configuration, the distally opened finger cot 200 is enclosed by the C-shaped housing 20,120.

In other words, the distally opened finger cot 200 is received in the reception volume V.

Beneficially, the C-shaped housing 20,120 and the distally opened finger cot 200 are configured such that the distally opened finger cot 200 is force-fitted to the C-shaped housing 20,120.

Alternatively, the C-shaped housing 20, 120 and the distally opened finger cot 200 are configured to form a snap-fit assembly.

The inner distally opened finger cot 200 is configured to encircle the finger's distal phalanx when the C-shaped housing 20 is placed around the fingertip.

This configuration maintains a relatively fixed orientation of the distal phalanx longitudinal axis P3 relative the first axis x and relative to the plane P in particular when the fingertip is force-fitted into the finger cot 200 removably fastened to the C-shaped housing 20.

This limits the latency time between the finger movement and the housing movement. In this way, the position of the electrical connection 25 is virtually unaffected by movements of the finger holding the phalanx.

The distally opened finger cot 200 encloses an inner space IV that the finger's distal phalanx when the C-shaped housing 20, 120 is placed around the fingertip.

The inner space IV is radially closed.

The inner space IV is delimited axially, along the longitudinal axis x by the distal aperture DA and the proximal aperture PA.

Beneficially, in an embodiment, the inner volume IV has a cylindrical part.

Beneficially, in an embodiment, the cylindrical part has an elliptical section. This shape closely fits a finger shape.

Beneficially, in an embodiment, the cylindrical part has a circular section.

The distal aperture DA and the inner surface IFC delimiting the inner space IV are configured and arranged such that the finger pad of the distal phalanx is in direct physical contact with the C-shaped housing 20,120 when the finger's distal phalanx P3 is encircled by the distally opened finger cot 200 when the distally opened finger cot 200 and the C-shaped housing 20, 120, are removably assembled as explained before while the fingertip is in abutment against the inner surface IFC, along the longitudinal axis xs of the distally opened finger cot 200.

Beneficially, in an embodiment, the distally opened finger cot 200 is configured such that the inner surface IFC encloses an inner space IV comprising cylindrical central part CP and a distal part DP in the extension of the central part CP along the longitudinal axis, the distal part DP partly closing the cylindrical central part CP and delimiting the distal aperture DA, such that the fingertip is able to come into abutment, along the longitudinal axis, against the part of the inner surface IFC when the fingertip is received in the inner space IV.

Beneficially, in an embodiment, the distally opened finger cot 200 closely fits the finger's shape.

It allows a precise guidance of the housing and the curved ultrasound transducer 30 by the finger.

Beneficially, in an embodiment, the distally opened finger cot 200 comprises a guide RA1, RA2 configured to guide the distally opened finger cot 200 such that the distally opened finger cot 200 slides along the guide relative to the C-shaped housing 20, 120 when assembling the distally opened finger cot 200 with the C-shaped housing 20, 120.

For example, the guide comprises two rails RA1, RA2.

Beneficially, in an embodiment, the rails RA1 and RA2 are configured and arranged be separated by the dorsal part 21 of the C-shaped housing 20 when assembling the distally opened finger cot 200 with the C-shaped housing 20.

This guides the dorsal part 21 along the rails RA1, RA2, facilitates the assembly of the C-shaped housing 20,120 and the distally opened finger cot 200. In other words, this design aids in inserting the distally opened finger cot into the volume or space enclosed by the C-shaped housing 20.

The finger cot 200 extends longitudinally along a longitudinal axis xs of the finger cot from a proximal end DV to a distal end PC of the finger cot 200.

In other words, each one of the two rails RA1, RA2 extends longitudinally along the longitudinal axis xs of the distally opened finger cot 200

Beneficially, in an embodiment, when the C-shaped housing 20, 120 and the distally opened finger cot 200 are assembled, the longitudinal axis xs of the distally opened finger cot 200 is parallel to the first axis x.

Beneficially, in an embodiment, the distally opened finger cot 200 has a shape that is substantially complementary to the shape of the C-shaped housing 20, 120, at least in the reference plane P when the distally opened finger cot 200 is removably fastened to the C-shaped housing 20.

This ensures a rigid connection between the C-shaped housing 20, 120 and the distally opened finger cot 200.

More specifically, the distally opened finger cot 200 comprises a central dorsal part CDP.

The distally opened finger cot 200 is configured such that the central dorsal part CDP fits the inner face 21T of the dorsal part 21 of the C-shaped housing 20, 120 when the C-shaped housing 20, 120 and the distally opened finger cot 200 are removably assembled.

Beneficially, in an embodiment, the distally opened finger cot 200 comprises a distal central part DF in the extension of the central dorsal part CDP, along the longitudinal axis xs, configured to abuts against the C-shaped housing 20, 120 along the first axis x when the C-shaped housing 20, 120 and the distally opened finger cot are assembled.

Beneficially, the distally opened finger cot 200 comprises a distal central part DF partially closes the central cylindrical part CP of the inner volume IV.

Beneficially, in an embodiment the distal central part DF is configured to abut against a part of an inner face 2IF of the C-shaped housing 20, 120 along the first axis x and to fit this part of an inner face 2IF of the C-shaped housing 20, 120 when the C-shaped housing 20, 120 and the distally opened finger cot are assembled.

For example, the distal central part DF is configured to abut against the upper inner face UT along the first axis and to have a shape complementary to the upper inner face UT to thit this upper inner face UT when the C-shaped housing 20, 120 and the distally opened finger cot are assembled.

Therefore, the distal central part DF is housed in the notch NR.

For example, the distal central part DF is delimited by a face having a circular section having a diameter sensitively equal to the diameter of the upper inner face UT.

The distal central part DF is intended to face the distal end of the fingernail of the finger encircled by the distally opened finger cot 200.

Alternatively, the distal central part DF is configured to abuts against the inner face CT of the central portion CE along the first axis and to fit the inner face CT of the central portion when the C-shaped housing 20, 120 and the distally opened finger cot are assembled.

It is for example the case when the inner face CT is contiguous to the inner face of the dorsal part 21T.

The distally opened finger cot 200 comprises two lateral parts L1 and L2 facing each other and enclosing the central dorsal part CDP and the distal central part DF.

The two lateral parts L1, L2, the central dorsal part CDP, the distal central part DF and the ventral part VO delimit (i.e. enclose) the inner volume IV.

The two lateral parts L1 and L2 extend continuously from the central dorsal part CDP to a ventral part VO configured to partly face and fit the ventral part VT of the C-shaped housing 20, 120 when the C-shaped housing 20, 120 and the distally opened finger cot are assembled.

For example, the lateral first part comprise the first rail RA1 and the second lateral part comprises the second rail RA2.

Beneficially, in an embodiment, each one of the rails RA1, RA2 protrudes along the longitudinal axis xs of the distally opened finger cot 200 from the distal central part DF when the distally opened finger cot 200 and the C-shaped housing 20 are assembled.

This feature ensures the guidance of the distally opened finger cot 200 along the C-shaped housing 20, 120 until the distal central part DF comes into abutments against the C-shaped housing 20, 120.

Beneficially, in an embodiment, a length of the distally opened finger cot 200 is designed such that the proximal end DV of the distally opened finger cot protrudes from the first proximal end PO1 and the second proximal end PO2 along the first axis x.

In other words, the length of the distally opened finger cot 200 is greater than the length of the reception volume V.

Beneficially, in an embodiment the length of the distally opened finger cot 200 is between 10 mm and 60 mm.

For example, the length of the distally opened finger cot is between 20 and 40 mm or between 25 and 35 mm.

Beneficially, in an embodiment a width of the distally opened finger cot measured along an axis parallel to the third axis when the distally opened finger cot is fastened to the C-shaped housing 20.

Beneficially, in an embodiment the width of the distally opened finger cot is between the between 16 mm and 30 mm. For example, it is between 18 mm and 25 mm.

The distally opened finger cot is for example made of a plastic material, such as a thermoplastic elastomer.

Beneficially, in an embodiment, a proximal end DV of the ventral part VO of the distally opened finger cot 200 proximally protrudes from the central dorsal part CDP, along the longitudinal axis xs of the distally opened finger cot 200.

In this configuration, the distally opened finger cot 200 encloses a recess RE intended to face the dorsal part of the finger's distal phalanx when the C-shaped housing 20, 120 surrounds the fingertip in abutment, along the longitudinal axis against the inner surface IFC.

In this configuration, the inner volume IV has, for example, a proximal part PP in proximal extension to the central part CP, the proximal part PP being a partially radially opened cylinder.

The proximal cylinder part PP is radially opened by the recess RE.

This facilitates the movement of the proximal phalanx of the finger relative to the distal phalanx of the finger.

Beneficially, the distally opened finger cot 200 is intended surrounds the finger's distal phalanx P2 without surrounding the second distal phalanx P2.

Beneficially, in an embodiment, the distally opened finger cot 200 is configured such that a maximal length of the inner volume IV along the longitudinal axis xs is comprises between 15mm or 200 and 30 mm or 35 mm.

In an example, a length measured along the longitudinal axis xs from the distal end of the distal central part DF to the proximal end PEP of central dorsal part CDP is equal to 20 mm.

Beneficially, in an embodiment, the distal central part DF distally protrudes from the ventral part VO of the distally opened finger cot 200 along the longitudinal axis xs. It allows a large direct physical contact surface of the finger pad with the C-shaped housing 20.

Beneficially, in an embodiment the ventral part VO comprises a first part VO1 facing the ventral part VT, IVT when the distally opened finger cot 200 and the C-shaped housing 20 are assembled and a second part VO2 distally protruding from the first part VO1 intended to be in direct physical contact with the inner face VI of the ventral part VT of the finger when the fingertip is received the distally opened finger cot 200 and is in abutment against the distal central part DF.

In an example, the first part VO1 and the second part VO2 are flat or substantially flat.

In an example, the first part VO1 is inclined relative to the face FO of the central dorsal part CDP intended to face the inner face 21T of the dorsal part 21 of the C-shaped housing 20.

This configuration is realized for example, such that the first part VO1 fits the inner face VI of the ventral part VT and the inner face 21 T of the dorsal part of the C-shaped housing 20 as shown in figure 1.

Beneficially, the second part VOS is parallel to the face FO of the central dorsal part CDP intended to face the inner face 21T of the dorsal part 21 of the C-shaped housing 20.

This configuration is compact.

### The curved ultrasound transducer

The curved ultrasound transducer 30 is configured to transmit and receive ultrasound pulses.

In an aspect, the curved ultrasound transducer is configured to transmit an ultrasound radiation toward a region of interest of a patient's tissues, for example a patient's face skin.

The ultrasound radiation is backscattered or reflected by the boundaries between different anatomic layers of tissue of the body or irregularities of the acoustic impedance in the tissue, toward the curved ultrasound transducer which receives the reflected ultrasound radiation and generates electrical signals based on the received reflected radiation.

The electrical signals are generated to allow for the reconstruction of an image, for example a bidimensional image, of the region of interest.

The electrical signals are for example transmitted from the probe to a remote treatment module.

The treatment module is beneficially configured to reconstruct the image of the region of interest using the electrical signals generated by the curved ultrasound transducer in response to the ultrasound radiation transmitted by the curved ultrasound transducer toward the region of interest or using data generated using the electrical signals generated by the curved ultrasound transducer.

The treatment module includes one or more electronic circuitries to carry its function of reconstructing the image of the region of interest. For example, the treatment module may include a data processor and a memory coded with instructions, which when executed by the data processor perform the tasks of image reconstruction.

The curved ultrasound transducer 30 is configured to transmit ultrasounds from the front curved face 31 toward the patient's skin and to receive ultrasound pulses along an axis. Such axis has a non-null component on a normal axis normal to the front curved face 31 of the curved ultrasound transducer 30 at a central position of the front curved face 31.

The curved ultrasound transducer 30 comprises a plurality of piezoelectric elements for transforming an electrical signal into an ultrasound pulse and vice et versa.

In an embodiment, the transducer comprises a plurality of piezoelectric elements that are adjacent to each other along a convex curve of a plane.

Beneficially, in an embodiment, the transducers are adjacent to each other along the convex external curve 31e formed by the front face 31 of the curved ultrasound transducer 30.

In another embodiment, the piezoelectric elements of the curved ultrasound transducer are arranged adjacent to each other along a plurality of curves that are three dimensionally arranged.

For example, the curved ultrasound transducer may comprise a first set of piezoelectric elements that are adjacent to each other along the convex external curve formed by the front curved face 31 of the curved ultrasound transducer 30 and a second set of piezoelectric elements that are also adjacent to each other along the convex external curve formed by the front curved face 31 of the curved ultrasound transducer 30. In this configuration, each piezoelectric element of the first set is adjacent to a piezoelectric element of the first set along an axis orthogonal to an axis orthogonal to the plane comprising the convex external curve.

The image is an image of the tissues inside the face and/or body of the patient.

The display of the image allows a physician to visualize the tissues beneath the patient's skin.

Beneficially, in an embodiment, the curved ultrasound transducer 30 is housed in the housing in such a way that the housing 20 encompasses all faces of the curved ultrasound transducer except for its the front face 31.

In an embodiment, a length of the convex external curve 31e is comprised between 15 and 45 mm.

The curved transducer 30 defines a field of view of the ultrasound fingertip probe. By "field of view" it is meant the zone in which the transducer 30 is intended to transmit and receive ultrasound pulses.

According to an embodiment, the field of view of the ultrasound fingertip probe is essentially contained in the reference plane P.

Beneficially, in an embodiment, the field of view of the ultrasound fingertip probe is contained within a sector having an angular aperture comprised between 10° and 90°.

For example, the angular aperture is between 20° and 50°, for example between 30° and 40° or between 40° and 50°.

Beneficially, in an embodiment, the curved ultrasound transducer is configured to transmit and receive ultrasound pulses in the domain of ultrasound sonic waves.

Beneficially, in an embodiment, the curved ultrasound transducer is configured to transmit and receive ultrasound pulses with a frequency between 10 and 20 MHz. These frequencies are suitable for visualizing human facial tissues at various shallow depths.

In an embodiment, the frequency is between 18 and 20 MHz. These frequencies are particularly well-suited for enabling high-precision imaging of both superficial and deep layers of facial skin, making them very convenient for assisting a physician during injection procedures in these tissues. In particular, they allow for the visualization of blood vessels in these tissues, helping to avoid them during injections.

Alternatively, the curved ultrasound transducer can be configured to transmit and receive ultrasound pulses with a frequency between 2.5 MHz and 10 MHz. These frequencies are suitable for visualizing tissues at greater depths, for example tissues of the abdominal region.

### Electrical connection

Beneficially, in an embodiment shown in figure 5, the ultrasound fingertip probe100 comprises a set of at least one electrical connection 25.

By "electrical connection", it is meant a set of at least one electrical wire, i.e an electrical cable, such as at least one coaxial cable.

At least one electrical connection is, for example, a flex cable.

At least one electrical connection is, for example, a flat cable.

At least one electrical connection is, for example, a coaxial cable.

Beneficially, in an embodiment, the set of at least one electrical connection 25 is encapsulated by an insulating sheath 26.

The ultrasound fingertip probe100 may comprise at least one electrical connection 25 that is arranged to provide electrical power to the curved ultrasound transducer 30 from a remote electrical power supply device to which the electrical connection is electrically connected.

Beneficially, in an embodiment, the electrical wire 25 extends continuously from the curved ultrasound transducer 30 to a proximal end of the electrical wire 25.

The curved ultrasound transducer 30 is housed in the C-shaped housing 20.

Therefore, the distal end of the electrical wire 25 is housed in the C-shaped housing 20.

Beneficially, in an embodiment, the dorsal part of the C-shaped housing comprises an aperture 27 through which the electrical wire 25 exits the C-shaped body.

Beneficially, in an embodiment, the aperture 27 is made in the first proximal end PO1.

Beneficially, in an embodiment, the aperture 27 is arranged such that the electrical wire 25 extends from the aperture 27 toward the proximal end of the electrical wire 25 along the dorsal part of the finger when the C-shaped housing 20 is placed around the fingertip.

For example, the aperture 27 is orthogonal to the first axis x.

These configurations reduce the inconvenience to the physician that could be caused by the electrical wire 25, for example when flexing their finger.

Another benefit of this configuration is the reduced risk of cable damage compared to a configuration where the electrical wire exits the C-shaped body from the second proximal extremity (facing the ventral face of the finger's distal phalanx). In such a configuration, the wire would extend longitudinally along the ventral part of the finger towards the proximal end of the electrical wire when the C-shaped body is placed around the fingertip.

Beneficially, in an embodiment, the aperture 27 is arranged in the C-shaped housing 20 such that the aperture 20 is crossed by an axis parallel to the first axis x.

Beneficially, in an embodiment, the aperture 27 is substantially orthogonal to the separation plane S.

By "substantially orthogonal", it is meant that an angle between the aperture 27 and the separation plane S may be less than 5 degrees, such as less than 3 degrees, or less than two degrees or less than one degree.

Beneficially, in an embodiment, the aperture 27 is crossed by the reference plane P.

For example, as shown in figure 5, the sheath 26 is attached to the C-shaped housing 20 by a tubular interface piece 28.

Beneficially, in an embodiment, the tubular interface piece 28 surrounds the sheath 26 and extends longitudinally along an axis xt, which is parallel to the x-axis and lies within the reference plane P. This ensures that the orientation of the sheath at the exit of the tubular interface piece 28 towards a proximal end of the sheath extends along and aligns with the axis xt.

Alternatively or additionally, the ultrasound fingertip probe100 comprises an energy storage device that is configured to provide electrical energy to the curved ultrasound transducer 30. The energy storage device may be housed in the C-shaped housing 20.

In an embodiment, at least one electrical wire is configured to transmit the electrical signals generated by the curved ultrasound transducer 30 to the treatment module.

Alternatively or additionally, the fingertip ultrasound comprises a wireless device configured to transmit wireless data representative of the electrical signals generated by the curved ultrasound transducer 30 to the treatment module. The wireless device is housed in the C-shaped housing.

According to an embodiment, the wireless device is configured to send date via a radio frequency communication protocol, such as via Wi-Fi or Bluetooth^{®} (a short-range wireless technology standard).

Beneficially, in an embodiment, the ultrasound probe 100 is wireless. In other words, the ultrasound probe 100 is configured to operate without being connected by any electrical wire to a remote device.

By "operate", it is meant to transmit and receive ultrasound pulses and to transmit electrical signals generated from the received ultrasound pulses to a remote treatment module. This wireless configuration allows the physician greater freedom of movement when manipulating the probe.

### Bracelet comprising a wire and straps receptacle, wire and straps

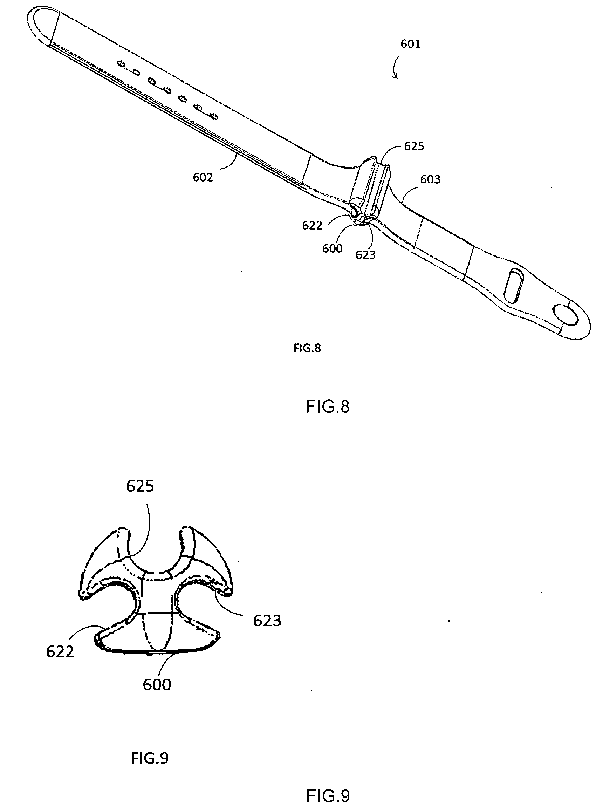

Beneficially, in an embodiment, the ultrasound probe comprises a wire and straps receptacle 600, as shown in Figures 8 and 9.

The wire and straps receptacle 600 is configured to removably receive two straps 602, 603 to form a bracelet or wristband 601 and to removably receive the at least one electrical wire 25 or the sheath 26 that receives the at least one electrical wire 25.

The bracelet or wristband 601 can be in an open configuration, where it extends globally longitudinally, as shown in Figure 8, or forms an open loop, or a closed configuration, where it forms a closed loop capable of radially enclosing the user's wrist.

The two straps 602, 603 are configured to move relative to each other such that the wristband switches from the open configuration to a closed configuration.

Beneficially, in an embodiment, in the closed configuration, the two straps 602, 603 are removably attached to each other.

The two straps comprise an attachment to attach the two straps 602, 603 to each other in the closed configuration.

Beneficially, in an embodiment, the attachment configured such that the size of the loop formed by the wristband 601 in the closed configuration is adjustable.

It allows for a snug fit around the physician's wrist regardless of its diameter within a predetermined range.

In an example, the attachment is a magnetic attachment or is a hoop and loop attachment.

The wrist and straps receptacle 600 comprise a wire receptacle 625 designed to removably accommodate the at least one electrical wire 25 or the sheath 26 containing the at least one electrical wire 25, and two lateral slots 622, 623, each intended to receive one of the two straps 602, 603 to form the wristband 601.

More specifically, each of the two lateral slots 622, 623 removably receive a first end of one of the two straps 602, 603.

Each of the two straps 602, 603 extends longitudinally from the first longitudinal end to a second end of the strap.

The second end of each strap is free in the open configuration.

The receptacle 600 is elongated along the longitudinal axis.

The wire receptacle 625, the first lateral slot 622 and the second lateral slot are distributed radially around a longitudinal axis of the receptacle 600.

Beneficially, in an embodiment, the receptacle 600 forms a one-piece element.

Beneficially, in an embodiment, the wire receptacle 625 delimits a substantially cylindrical volume or space in which the at least one electrical wire 25 or the sheath 26 is removably inserted.

Beneficially, in an embodiment, when it is received in the wire receptacle 661, the at least one electrical wire 25 or the sheath 26 is able to slide relative to the receptacle 600 along the wire receptacle 625.

This configuration allows for placing the wristband 601 in a desired position along the user's wrist while in the open configuration, and then switching to the closed second configuration to completely and snugly enclose the physician's wrist radially.

Beneficially, in an embodiment, the wire receptacle 661 is elastically deformable from a first configuration, in which the wire can be inserted into the wire receptacle 661, to a second configuration, in which the wire 25 or the sheath 26 is radially enclosed by the receptacle 600 in the wire receptacle 625 in a snug manner, ensuring that the wire 25 or the sheath 26 is securely held in the wire receptacle 625.

Beneficially, in an embodiment, each of the two lateral slots 622, 623 is elastically deformable from a first configuration in which the corresponding strap 602, 603 can be inserted into the lateral slot 622, 623, to a second configuration, in which the strap 602, 603 is radially enclosed by the receptacle 600 in the lateral slot 622, 623 in a snug manner, ensuring that the strap 602, 603 is securely held in the lateral slot 622, 623.

Moreover, when the user's wrist is snugly received in the closed loop formed by the wristband in its closed configuration, the radial position of the at least one electrical wire 25 or sheath 26 relative to the user's wrist around a longitudinal axis along which the wrist of the user is elongated is fixed.

Beneficially, in an embodiment, the wristband 601 is arranged relative to the at least one electrical wire 25 or the sheath 26 and the C-shaped housing 20 to be able to attach the at least one electrical wire 25 or the sheath 26 to the wrist in a position where the electrical wire 25 or sheath extends longitudinally along a dorsal face of the wrist.

Beneficially, in an embodiment, at least one of the wire receptacle 625, the first lateral slot 622 and the second lateral slot 623 is substantially cylindrical.

The wristband allows for fast, easy and sturdy placement of electrical wire 25 or of the sheath 26 along the dorsal face of the wrist of the user.

### System

An aspect of the invention further relates to a system comprising the ultrasound probe, the treatment module, the optional wireless device, a display.

The curved ultrasound transducer 30 is configured to acquire ultrasound pulses during an acquisition step and to generate electrical signals from the acquired ultrasound pulses.

In an embodiment, during the acquisition step, the curved ultrasound transducer 30 is pressed against the patient's skin such that an image generated using signals or data representative of the electrical signals depicts or shows the patient's subcutaneous tissues.

Beneficially, in an embodiment, the commands are generated such that the curved ultrasound transducer 30 electronically scans the region of interest during the acquisition step.

The ultrasound probe optionally comprises a wireless device configured to generate wireless data and transmit wirelessly the wireless data to the treatment module.

Alternatively, at least one electrical wire is configured to transmit electrical signals to the treatment module.

The treatment module is configured to computer-generate an image of the zone of interest using the electrical signals or the wireless data.

The display is configured to display the image. The displayed image assists the physician during an injection procedure by helping to avoid blood vessels, thus making the procedure safer for the patient.

### Injection or positioning procedure

An aspect of the invention further concerns a method for injecting a substance beneath the patient's skin. The substance injected can be a fluid, such as a treatment fluid. This substance can also be any possible filler, regardless of whether the filler is in a fluid or gel form or any other form.

The method may concern the positioning of any medical device beneath the patient's skin, such as the placement of a thread.

In an embodiment, the method for injecting a substance and/or positioning a medical device uses the ultrasound hand probe according to an aspect of the invention.

In an embodiment, the method for injecting a substance and/or a medical device under the skin of a patient comprises the following steps:
- Optionally removably fastening a finger retainer to the C-shaped housing 20 or the C-shaped part of the ultrasound fingertip probe,
- placing the C-shaped body around the fingertip,
- acquiring ultrasound pulses from a zone of interest of a patient using the curved ultrasound transducer 30, the curved ultrasound transducer 30 being pressed against the patient's skin,
- generating an image of the zone of interest using ultrasound pulses received by the curved ultrasound transducer 30 when acquiring the ultrasound pulses,
- displaying the image on a screen,
- injecting, via the syringe, a treatment liquid beneath the skin of the patient or positioning a medical device beneath the skin of the patient.

### Applications

According to an embodiment, the ultrasound probe or the ultrasound system is used to assist a physician during an aesthetic procedure.

For instance, it can help the physician visualize the injection site for aesthetic medications.

According to an embodiment, the ultrasound system is used to assist a physician during angiology procedures.

For instance, it helps the physician to visualize the path of blood vessels on every part of the body of a patient.

According to an embodiment, the ultrasound system is used to assist a physician during an emergency medicine procedure. By this way it can help the physician visualize hidden parts of the patient's body during urgent procedures.

According to an embodiment, the ultrasound system is used to help the physician visualize hemodynamic, muscles, various anatomical planes beneath the skin, for example from the skin to a bone, any foreign bodies or fat tissue already injected, needles and cannulas required for injection or any type of injection devices. It also helpful when the operator needs to inject a product to dissolve an existing foreign body or fat tissue.

According to an embodiment, the ultrasound system is used to help the physician visualize the female and male urinary tract during urology procedures.

According to an embodiment, the ultrasound system is used to help a physician during medical procedures or interventions on the penis or the scrotum.

According to an embodiment, the ultrasound system is used to help a physician during any rheumatology procedures, mainly any type of intra-articular injections.

According to an embodiment, it can help the physician visualize articulations to treat any musculoskeletal issues.

According to an embodiment, the ultrasound system is used to help a physician during sport medicine and orthopaedic procedures. A benefit is to obtain precise images of body parts to aid in procedures.

According to an embodiment, the ultrasound system is used to help a physician to visualize any organs dedicated to the endocrinology procedures by providing him ultrasound images of the patient's body.

According to an embodiment, the ultrasound system is used to help a physician intended to assist medical and surgical examinations and treatment of all medical specialties exploring the cavities of the body such as gynecology or otorhinolaryngology.

According to an embodiment, the ultrasound system can be applied in veterinary medicine.

According to an embodiment, the ultrasound system is used to help a physician during neurosurgery procedure by providing him with ultrasound images of the patient's body.

According to an embodiment, the ultrasound system is used to help a physician during an ophthalmology procedure and/or treatment.

In all these applications, the ultrasound system provides valuable visual information to help physicians perform procedures more accurately and safely

The articles "a" and "an" may be employed in connection with various elements and components of compositions, processes or structures described herein. This is merely for convenience and to give a general sense of the compositions, processes or structures. Such a description includes "one or at least one" of the elements or components. Moreover, as used herein, the singular articles also include a description of a plurality of elements or components, unless it is apparent from a specific context that the plural is excluded.

It will be appreciated that the various embodiments and aspects of the inventions described previously are combinable according to any technically permissible combinations. For example, various aspects of the present disclosure may be used alone, in combination, or in a variety of arrangements not specifically described in the embodiments described in the foregoing and is therefore not limited in its application to the details and arrangement of components set forth in the foregoing description or illustrated in the drawings. For example, aspects described in one embodiment may be combined in any manner with aspects described in other embodiments.

The present invention has been described and illustrated in the present detailed description and in the figures of the appended drawings, in possible embodiments. The present invention is not however limited to the embodiments described. Other alternatives and embodiments may be deduced and implemented by those skilled in the art on reading the present description and the appended drawings.

In the claims, the term "includes" or "comprises" does not exclude other elements or other steps. The different characteristics described and/or claimed may be beneficially combined. Their presence in the description or in the different dependent claims do not exclude this possibility. The reference signs cannot be understood as limiting the scope of the invention.

In the specification, the proximal and distal parts are defined along the first axis x or along the axis xs or xi.

## Claims

1. An ultrasound fingertip probe comprising:
- a curved ultrasound transducer (30) configured to transmit and receive ultrasounds,
- a C-shaped housing (20) intended to surround a fingertip of a user, the C-shaped housing having a C-shape in a reference plane P, the curved ultrasound transducer (30) being housed in the C-shaped housing (20),
- a finger retainer (110, 200) removably fastened to the C-shaped housing (30) such that the finger retainer limits a tilt of the finger relative to the reference plane (P) when the C-shaped housing (20) surrounds the fingertip.

2. The ultrasound fingertip probe as claimed in claim 1, wherein the finger retainer is a distally opened finger cot (200) removably fastened to the C-shaped housing (20) to receive the finger's distal phalanx when the C-shaped housing (20) surrounds the fingertip.

3. The ultrasound fingertip probe according to the preceding claim, wherein the distally opened finger cot (200) comprises a guide configured to guide the C-shaped housing along the distally opened finger cot (200) when removably assembling the distally opened finger cot (200) with the C-shaped housing (20).

4. The ultrasound fingertip probe as claimed in claim 1, wherein the finger retainer (110) comprises two wings facing each other, the reference plane (P) being interposed between the two wings.

5. The ultrasound fingertip probe as claimed in any one of claims 1 to 4, wherein the C-shaped housing (20) is configured to remain free from distortion when being placed around the fingertip.

6. The ultrasound fingertip probe as claimed in any one of claims 1 to 5, wherein the C-shaped housing (20) comprises:
- a dorsal part (21) configured to face a dorsal part of the fingertip when the C-shaped housing surrounds the fingertip, the dorsal part extending continuously from a first proximal end (PO1) to a curved part (22) of the C-shaped housing (20),
- the curved part (22) extending continuously from the dorsal part (21) to a second proximal end (PO2) and facing a ventral part of the fingertip when the C-shaped housing surrounds the fingertip while the dorsal part (21) faces the dorsal part of the fingertip,
a separation plane (S) being interposed between the first proximal end (PO1) and the second proximal end (PO2), the separation plane being orthogonal to the reference plane (P) and including a first axis (x) of the reference plane (P) along which a longitudinal axis of the finger's distal phalanx extends when the C-shaped housing (20) surrounds the fingertip.

7. The ultrasound fingertip probe (10) as claimed in claim 6, wherein an inner face (21T) of the dorsal part (21) forms a straight line in, the reference plane (P), the inner face of the dorsal part being intended to face the finger's the distal phalanx when surrounds the fingertip.

8. The ultrasound fingertip probe as claimed in anyone of claims 6 to 7, wherein the curved ultrasound transducer (30) being housed in the curved part (22) such that a front curve face (31) of the curved ultrasound transducer (30) forms, in the reference plane (P), a convex front curve (31e) extending from a proximal point (pt) to a distal point (dt), towards the dorsal part (21) of the C-shaped housing (21) along a second axis (y) of the reference plane (P) orthogonal to the first axis (x), a value of an angle formed by a tangent (T) of the convex front curve (31e), at predetermined point of the convex front curve (31e), with the first axis x, continuously increases with a position of the point along the convex front curve (31e) from the proximal point (pt) to the distal point (dt).

9. The ultrasound fingertip probe as claimed in anyone of claims 6 to 8, wherein a central portion (CE) of an inner face (CT) of the curved part (22), forms, in the reference plane (P), a curve having a first radius of curvature, the inner face (31) of the central portion (CE) being interposed, along an axis parallel to the first axis (x) in the reference plane (P), between the front face (31) of the curved ultrasound transducer (30) and a reception volume (V) receiving the fingertip when the C-shaped housing surrounds the fingertip, the inner face of the curved part being intended to face the ventral part of the finger's distal phalanx when the C-shaped housing surrounds the fingertip such that the dorsal part of the C-shaped housing faces the dorsal part of the finger's distal phalanx.

10. The ultrasound fingertip probe as claimed in any one of claims 1 to 9, wherein a part of an outer face (1EF) of the C-shaped part (10) of the ultrasound fingertip probe delimits and encloses the front curved face (31) of the curved ultrasound transducer (30), and the front curved face (31) of the curved ultrasound transducer (30) form together a continuous convex face that is substantially a partial cylinder, having a generatrix of the cylinder being orthogonal to the reference plane (P).

11. The ultrasound fingertip probe as claimed in anyone of claims 8 to 10, wherein the front face (31) of the curved ultrasound transducer (30) is distant along the second axis (y) of the reference plane (P) from a reference point (pr) of the curved part (22), the reference point (pr) being the farthest from the separation plane (S) along the second axis (y) among the points of the curved part (22).

12. The ultrasound fingertip probe as claimed in anyone of the preceding claims, wherein the curved part (22) of the C-shaped housing (20) comprises a ventral part (VT) comprising the second proximal end (PO2), the ventral part (VT) being delimited by an outer face (VE) of the ventral part (V) and by an inner face (VI) interposed between the outer face (VE) and a reception volume (V) delimited and surrounded by the C-shaped housing (20), the dorsal part (21) being delimited by an outer face (21E) of the dorsal part (21) and by an inner face (21T) of the dorsal part (21) interposed between the outer face (21E) of the dorsal part (21) and the reception volume (V), the outer face (VE) of the ventral part (VT) being parallel to the outer face (21E) of the dorsal part (21).

13. The ultrasound fingertip probe according to any one of the preceding claims, wherein the C-shaped housing (20) comprises a notch (NR) configured and disposed to receive a distal end of a fingernail protruding, along the first axis, from the pad of the finger's distal phalanx when the C-shaped housing surrounds the fingertip, the dorsal part of the C-shaped housing (20) facing a dorsal part of the finger.

14. The ultrasound fingertip probe as claimed in anyone of the preceding claims, comprising a wristband (601) comprising a receptacle (600) comprising a wire receptacle (625) receiving an electrical wire (25) or a sheath receiving a plurality of electrical wires (25) in a removable manner, and two slots (222, 223) removably receiving two straps (602, 603), the wristband (601) being configured to be able to be in an open configuration in which the wristband extends substantially longitudinally and in a second configuration in which the wristband forms a closed loop.

15. A kit comprising a ultrasound fingertip probe as claimed in any one of claims 1 to 14 and a plurality of finger retainers (110, 200) configured to be able to be removably fastened to the C-shaped housing (20) to limit tilt of a longitudinal axis of the finger relative to the reference plane (P) when the C-shaped housing (20) surrounds the fingertip, the plurality of finger retainers comprising a plurality of distally opened finger cots (200) enclosing spaces having different diameters and / or the plurality of finger retainers having two wings facing each other, the two wings of different retainers being separated by respective distinct maximal distances.
